Europäisches Patentamt

European Patent Office

Office européen des brevets

(19)

(11) **EP 0 996 431 B1**

(12) **EUROPEAN PATENT SPECIFICATION**

(45) Date of publication and mention
of the grant of the patent:
**31.03.2004 Bulletin 2004/14**

(51) Int Cl.[7]: **A61K 31/01**, A61K 31/355
// (A61K31/01, 31:355)

(21) Application number: **98928504.4**

(22) Date of filing: **18.06.1998**

(86) International application number:
**PCT/IL1998/000286**

(87) International publication number:
**WO 1998/057622 (23.12.1998 Gazette 1998/51)**

(54) **SYNERGISTIC COMPOSITIONS OF LYCOPENE AND VITAMIN E FOR THE PREVENTION OF LDL OXIDATION**

SYNERGISTISCHE ZUSAMMENSETZUNGEN VON LYCOPIN UND VITAMIN E, DIE DER OXIDATION DER LDL VERHINDERN

COMPOSITIONS SYNERGIQUES POUR LYCOPENE ET VITAMINE E PERMETTANT LA PREVENTION D'OXYDATION DE LIPOPROTEINE DE FAIBLE DENSITE (LDL)

(84) Designated Contracting States:
**AT BE CH CY DE DK ES FI FR GB GR IE IT LI LU MC NL PT SE**

(30) Priority: **19.06.1997 IL 12111297
16.06.1998 IL 12493098**

(43) Date of publication of application:
**03.05.2000 Bulletin 2000/18**

(73) Proprietors:
• **Lycored Natural Products Industries Ltd
84102 Beer Sheva (IL)**
• **Fuhrman, Bianca
Haifa 32983 (IL)**
• **Aviram, Michael
Kiryat Haim 26253 (IL)**

(72) Inventors:
• **FUHRMAN, Bianca
32983 Haifa (IL)**
• **AVIRAM, Michael
26253 Kiryat Haim (IL)**
• **NIR, Zohar
85025 Meitar (IL)**
• **ZELKHA, Morris
84965 Omer (IL)**

(74) Representative: **Modiano, Guido, Dr.-Ing. et al
Modiano, Josif, Pisanty & Staub,
Baaderstrasse 3
80469 München (DE)**

(56) References cited:
WO-A-92/05780          WO-A-95/35099
WO-A-96/19215          WO-A-96/19217

• FUHRMAN B. ET AL: "Tomato lycopene and beta carotene inhibit low density lipoprotein oxidation and this effect depends on the lipoprotein vitamin E content" NUTR. METAB CARDIOVASC. DIS., vol. 7, 1997, pages 433-443, XP002082418 cited in the application
• STAHL W. ET AL: "Carotenoid mixtures protect multilamellar liposomes against oxidative damage: Synergistic effects of lycopene and lutein" FEBS LETTERS, 1998, 427/2 (305-308), XP002082207 Netherlands
• FUHRMAN B. ET AL: "Hypocholesterolemic effect of lycopene and b-carotene is related to suppression of cholesterol synthesis and augmentation of LDL receptor activity in macrophages" BIOCHEM BIOPHYS RES COM, vol. 233, 1997, pages 658-662, XP002082199 cited in the application
• WOODALL A.A. ET AL: "Carotenoids and protection of phospholipids in solution or in liposomes against oxidation by peroxyl radicals: Relationship between carotenoid structure and protective ability" BIOCHIMICA ET BIOPHYSICA ACTA - GENERAL SUBJECTS, 1997, 1336/3 (575-586), XP002082208 Netherlands

- WISE J.A. ET AL: "Changes in plasma carotenoid, alpha-tocopherol, and lipid peroxide levels in response to supplementation with concentrated fruit and vegetable extracts: A pilot study" CURRENT THERAPEUTIC RESEARCH - CLINICAL AND EXPERIMENTAL, 1996, 57/6 (445-461), XP002082209 USA

- HAILA KATRI M._(A): "Effects of lutein, lycopene, annatto, and gamma-tocopherol on autoxidation of triglycerides." JOURNAL OF AGRICULTURAL AND FOOD CHEMISTRY, 1996, XP002082742

## Description

### Field of the Invention

The present invention concerns a composition containing lycopene and Vitamin E.

This present invention more particularly relates to a synergistic mixture of lycopene and vitamin E and its use in the prevention of LDL oxidation, and preparing pharmaceutical and dietary compositions for arresting the progression of atheroclerosis.

### Background of the Invention

Atherosclerosis is the major single cause of mortality in western society. It is assumed to reach up to 50 % of all mortality (*Davies MJ, and Woolf N. Atherosclerosis: what is it and why does it occur? Br Heart J 69:S3, 1993*). In addition, it results in significant cardiac morbidity, such as anginal syndromes, myocardial infarctions, ischemic cardiomyopathy, sudden cardiac death, cerebrovascular accidents, and peripheral vascular disease. Indisputable evidence for an association between coronary heart disease (CHD) and risk factors, such as arterial hypertension, cigarette smoking and hyperlipidemia, has been derived from a variety of epidemiological studies. Of all the risk factors established thus far, lipid disorders play a key role in the pathogenesis of atherosclerotic vascular disease, especially of CHD. Many epidemiological and clinical trials have demonstrated the powerful association between hyperlipidemia and the widespread incidence of CHD. The Framingham Heart Study (*Castelli WP*, *Anderson K.*, *Wilson PW.*, *Levy D. Lipids and risk of coronary heart disease. The Framingham Study. Ann Epidemiol 2(1-2): 23-28, 1992*), which has been continuous since 1984, showed that hypercholesterolemia is a major contributor to the development of CHD. The link between atherosclerosis and cholesterol has been confirmed by a number of clinical trials.

Fats are insoluble in the aqueous medium of the blood. Thus, transport of the lipids triglycerides, phospholipids and cholesterol occurs exclusively by way of lipid-protein complexes, the lipoproteins. The lipoproteins are classified into 4 broad classes-chylomicrons, very low density lipoprotein (VLDL), low density lipoprotein (LDL), and high density lipoprotein (HDL), which differ in their composition, size, and potential atherogenicity.

Measurement of total cholesterol level in plasma reflects the sum of cholesterol being transported in each individual lipoprotein. However, LDL and HDL are the main cholesterol carriers in plasma, and only a small fraction of cholesterol is carried in VLDL or in the chylomicrons. Atherosclerosis is a gradual pathological process, which is characterized by an accumulation of lipid filled macrophages (foam cells), and smooth muscle cells resulting in lesions that thicken and harden the arterial wall. The main source for the cholesterol accumulating in the foam cells is the circulating LDL. There is evidence from numerous epidemiological and clinical studies that LDL, as the carrier of ca. 70% of the total cholesterol in plasma, are the most potent atherogenic lipoproteins. Their elevation carries a particular risk, and reduction in LDL cholesterol constitutes a diminished atherosclerotic risk. The Lipid Research Clinics Coronary Primary Prevention Trial (LRC-CPPT) had for the first time presented firm proof that in man, a lowering of LDL cholesterol level reduces the rate of myocardial infarction and infarction mortality.

Nearly all cells, including macrophages, take up exogenous cholesterol via LDL-receptors. Increase in cell cholesterol content, however, results in down-regulation of LDL receptor number, thereby protecting cells from excessive accumulation of cholesterol by way of this pathway. It was shown' that chemical or biological modification, including oxidation of LDL, results in increased uptake of the modified lipoprotein by way of other cell surface receptors, termed scavenger receptors. These receptors are present on macrophages and endothelial cells.

Oxidative modification of LDL is thought to play a causal role in atherosclerosis: (see e.g. Steinberg D., Parthasarathy S., Carew T.E., Khoo J.C. and Witztum J.L, "Beyond cholesterol: modifications of low-density lipoprotein that increase its atherogenicity", N. Engl. J. Med. 1989; 320: 915-924; Haberland M.E. and Fogelman A.M., "The role of altered lipoproteins in the pathogenesis of atherosclerosis", Am. Heart. J. 1987; 113: 573-577; and Witztum J.L., "The oxidation hypothesis of atherosclerosis", Lancet. 1994; 344, 793). It is believed, accordingly, that prevention of LDL oxidation by antioxidants may arrest the progression of atherosclerosis. Aviram M. Beyond cholesterol: Modification of lipoproteins and increased atherogenicity. In Atherosclerosis, inflammation and thrombosis (G.G. Neri Serneri, G.F. Gensini, R. Abbate and D. Prisco eds)-Scientific Press-Florence, Italy, pp: 15-36, 1993.

The ability to prevent the development of atherosclerotic lesions would have major implications for the public health. Thus using therapeutic agent with plural effects, such as lowering cholesterol and inhibiting oxidative modification, might have beneficial effects over other individual agent.

Carotenoids are colored pigments with lipophilic properties, widely distributed in fruits and vegetables, (e.g. β-carotene in carrots and lycopene in tomatoes) and possess some antioxidant properties: (see e.g. Burton G.W., "Antioxidant action of carotenoids," 1989; J. Nutr. 119:109-111 and Krinsky N.I., "Antioxidant functions of carotenoids", Free Radic. Biol. Med. 1989, 7: 617-635 (9-13)). Carotenoids are transported within circulating lipoproteins, and it was postulated that they participate in the protection of LDL from oxidative modification.

Carotenoids consumption was shown in previous epidemiological studies to be associated with reduced cardiovascular mortality (see e.g.: Kohlmeier L. and Hasting S.B., "Epidemiologic evidence of a role of carotenoids in cardiovascular disease prevention", Am. J. Clin. Nutr. 1995; 62: 137S-146S), although recent data did not demonstrate similar beneficial effect (see e.g.: Hennekens C.H., Buring J.E., Manson J.E., Stampfer M, Rosier B., Cook N.R., Belanger C., LaMotte F., Gaziano J.M., Ridker P.M., Willett W. and Peto R., "Lack of effect of long-term supplementation with beta carotene on the incidence of malignant neoplasms and cardiovascular disease", N. Engl. J. Med. 1996; 334: 1145-1149).

Reduced plasma lipid peroxidation was recently shown to be associated with increased consumption of tomatoes. Low levels of plasma carotenoids were shown to be associated with an increased risk of myocardial infarction, and recently it was demonstrated that the association between $\beta$ -carotene and acute myocardial infarction depends on the polyunsaturated fatty acids status, and that feeding the all-trans isomer of $\beta$ -carotene to cholesterol-fed rabbits attenuated the extent of their atherosclerosis, with no effect on LDL oxidizability ex vivo. Data on the ability of $\beta$ -carotene supplementation in vitro or in vivo to protect LDL from oxidation are conflicting: some studies found an inhibitory effect of $\beta$ -carotene supplementation on LDL oxidation, whereas several other did not find such effect.

Lycopene, the open chain analog of $\beta$ -carotene, shares with it similar structure with an extended conjugated double bonds. In human plasma, lycopene and $\beta$ -carotene are quantitatively the major carotenoids. Lycopene was shown to possess the greatest quenching ability of singlet oxygen among the various carotenoids (DiMascio P., Kaiser S. & Sies H., "Lycopene as the most efficient biological carotenoid singlet oxygen quencher," Arch. Biochem. Biophys., 1989, 274: 532-538) and it was shown to be at least twice as effective antioxidant as $\beta$ -carotene in protecting blood lymphocytes form $NO_2$ radical damage (Bohm F., Tinkler J.H., and Truscott T.G., "Carotenoids protect against cell membrane damage by the nitrogen dioxide radical", Nature Medicine, 1995, 2: 98-99).

We have recently demonstrated a protective effect of tomatoes lycopene against oxidative modification of LDL. This protection of LDL by lycopene exceeds the protection exhibited by $\beta$-carotene, was selective only to LDL's with high vitamin E content and was shown when the carotene was present in combination with vitamin E. (see e.g., Fuhrman B, Ben Yaish L, Attias J. Hayek T. Aviram M. *Tomatoes lycopene and $\beta$-carotene inhibit low density lipoprotein oxidation and this effect depends on* the *lipoprotein vitamin E. content.* Nutr Metab. Cardiovasc. Dis 7: 433-443, 1997). Furthermore, we have also demonstrated that dietary supplementation of lycopene acts as moderate hypocholesterolemic agent secondary to its inhibitory effect on cellular cholesterol synthes (see e.g. Fuhrman B., Elis A, Aviram M., *Hypocholesterolemic effect of lycopene and $\beta$-carotene is related to suppression of cholesterol synthesis and augmentation of LDL receptor activity in macrophages.* Bichem. Biophys. Res. Commun. 233: (658-662,1997).

Israel Patent Application No. 123,132 recently filed covers the use of a mixture of lycopene and garlic to be used in preventing LDL oxidation.

WO 96/19217 discloses pharmaceutical compositions comprising lycopene and alphatocopherol and the use of these compositions for reducing seruim level of cholesterol and lipids.

WO 92/05780 discloses a method for inhibiting the occurrence of a major vascular event in a subject by administration of beta-caroten, or analogs of beta-caroten as lycopene, and/or vitamin E, either alone or in any combination.

### Objectives

It is an objective of the present invention to provide a novel mixture of lycopene and vitamin E. It is an objective of the present invention to provide a synergistic mixture of lycopene and vitamin E active in blocking the oxidation of LDL and/or atherosclerosis and reduce the cholesterol levels in plasma. An additional objective of the present invention is the use of the above described mixture to prepare a pharmaceutical or dietary composition for arresting the progression of atherosclerosis. A further objective of the present invention is to provide a composition for the arresting of the progression of atherosclerosis containing essentially products acceptable and desertable of the harm diet.

### Summary of the Invention

The invention is based on the discovery that there is an unexpected and surprising synergistic effect between lycopene and vitamin E, in the prevention of LDL oxidation. Lycopene is a natural product and is present in tomatoes in various concentrations, but can also be produced by fungal, algal or by fermentation, in genetically modified organisms (GMO) or by synthetic method.

A widely used source of vitamin E is soybean distillate.

The use of combinations of lycopene and vitamin E as medicines or components of pharmaceutical compositions or of dietary or health compositions for the prevention of LDL oxidation is an aspect of the present invention.

The compositions may contain other dietary components, additives, excipients, binding agents, coatings, etc., or other compounds that have no significant effect in preventing LDL oxidation. The compositions used in the invention, in view of their purpose, are pharmaceutical compositions. However, since their components are individually acceptable ingredients of the human diet, and in fact are individually present in foodstuffs, they may be considered and used as dietary or health supplements.

The use of tomato oleoresin for the prevention of LDL oxidation is a specific aspect of the present invention.

The compositions used i n the invention should preferably comprise lycopene and vitamin E in a molar proportion of 1:400 to 10:1, preferably 1:80 to 5:1. The amount of lycopene in each unit of the composition, e.g. a tablet or soft gel capsule, should be in the range of 1 - 4, preferably about 2 - 3 and up to 15 mg of lycopene.

These compositions are useful for arresting the progression of atherosclerosis, by administration to a patient lycopene and vitamin E, in the aforesaid relative amounts.

The invention comprises a method of preventing LDL oxidation in vitro.

### Detailed Description of Preferred Embodiments

In a preferred way of carrying out the invention, and in the Examples that will be described, lycopene was obtained from tomato oleoresin. The oleoresin was supplied by LycoRed-Natural Products Industries, Ltd, Beer Sheva, Israel. It consisted of crystalline lycopene (6.0%) suspended in the lipid phase of the tomato. Fatty acids as triglycerides constituted the major part (72%) of the oleoresin, whereas 19% were non-saponified materials including 6.0% lycopene, 0.1% $\beta$-carotene and 1% vitamin E. The rest consists of water and water soluble materials. The lycopene used was extracted and purified mainly in the all-trans configuration. Lycopene can be extracted from tomato oleoresin, as described in Patent Application No. WO 97/48287.

Vitamin E is readily available in the pharmaceutical market.

Said ingredients are conveniently available and used in the form of oleoresin or in a solid form such as beadlets.

### MATERIALS

#### Supplementation of human LDL with lycopene

Stock solutions of 1mM of purified lycopene or of 10mg/ml of tomato's oleoresin, were prepared in Tetrahydrofuran (THF, HPLC grade). All procedures were performed in dim light. LDL was incubated with the respective concentrations of tomato's oleoresin or with 3$\mu$M of lycopene (3$\mu$l/ml THF, derived from a stock solution of 1mM) for 30 minutes at 37°C in the dark. LDL incubated with THF alone served as control.

#### LDL oxidation

Oxidation of LDL was carried out in a shaking water bath at 37°C under air, in plastic tubes (1cm in diameter). For metal ions dependent oxidation LDL was incubated for 4 hours at 37°C with freshly prepared $CuSO_4$ (5$\mu$M).

LDL oxidation was determined by measuring the amount of thiobarbituric acid reactive substances (TBARS) (See: Buege J.A. and Aust S.D. Microsomal lipid peroxidation. Methods Enzymol. 1978; 52: 302-310) and by lipid peroxides formation using a commercially available kit.

#### Effects of lycopene or vitamin E alone against LDL oxidation

LDL(100 $\mu$g of protein/ml) was preincubated for 30 min at 37°C with either vitamin E or lycopene in various forms. LDL oxidation was induced by the addition of 5$\mu$M of $CuSO_4$ and was preincubated for 30 min at 37°C. LDL oxidaton was measured by the TBARS assay or by measuring the inhibition of the formation of lipid peroxides. Results are shown in Figure 1 for vitamin E inhibition and Figure 2 for crystalline lycopene. Figure 3 shows the effect for tomato oleoresin, and for comparison, Figure 4 shows the results for tomato oleoresin where the natural occurring vitamin E was extracted out.

#### Effect of lycopene in combination with vitamin E against LDL oxidation

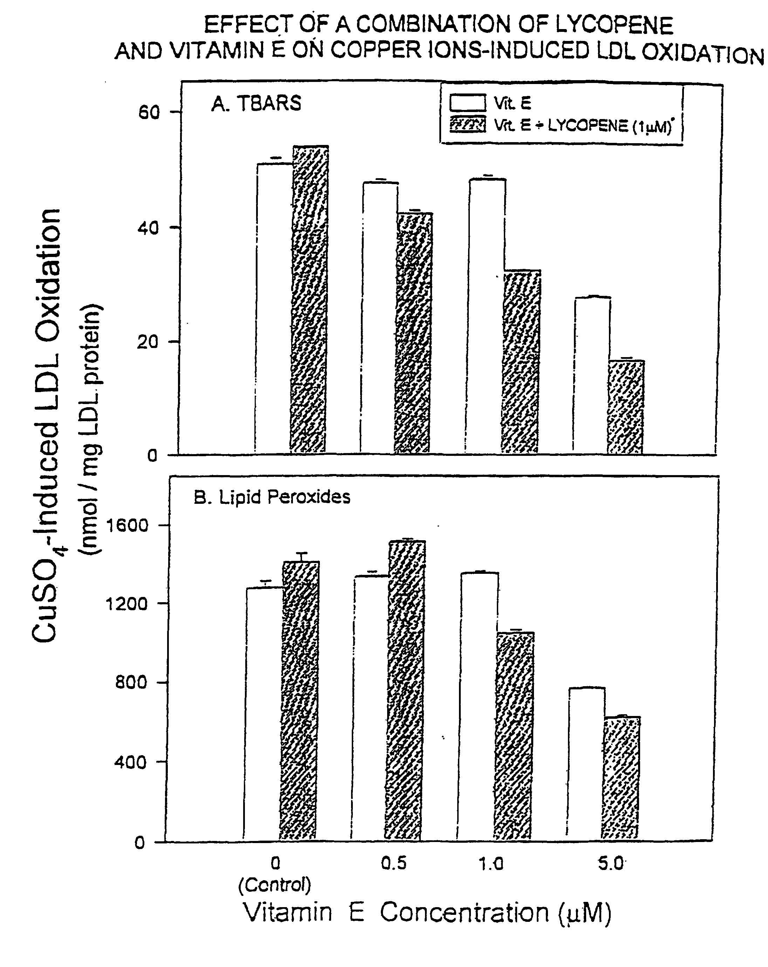

The antioxidative effect of a combination of vitamin E with lycopene against LDL oxidation, was assessed is a manner illustrated by Figs. 5 to 7, in which the degree of inhibition of LDL oxidation by individual anti-oxidants is shown in the ordinate.

The tests hereinbefore described show that when vitamin E and lycopene are added together to LDL during copper-induced oxidation, a synergistic effect occurs. This synergistic inhibitory effect on LDL oxidation, is believed to be probably related to the contribution of vitamin E and lycopene to different antioxidative mechanisms. Whereas vitamin E is a chain breaking agent and acts as a free radicals scavenger, lycopene is a potent free radical scavenger as well as an oxygen quencher. Therefore, administration of a combination of these two antioxidants with synergistic inhibitory effects on LDL oxidation, such as vitamin E and lycopene, appears to be beneficial over the administration of individual antioxidants separately. This is further evidenced by the inhibitory effect on LDL oxidation of tomato's oleoresin, which contains a combination of antioxidants..

In some cases as published in the literature, vitamin E alone does not efficiently lower the LDL oxidation. Nevertheless, when lycopene or the oleoresin of tomatoes is added to the vitamin E. a marked synergistic lowering of the LDL oxidation occurs.

EXAMPLES

GENERAL

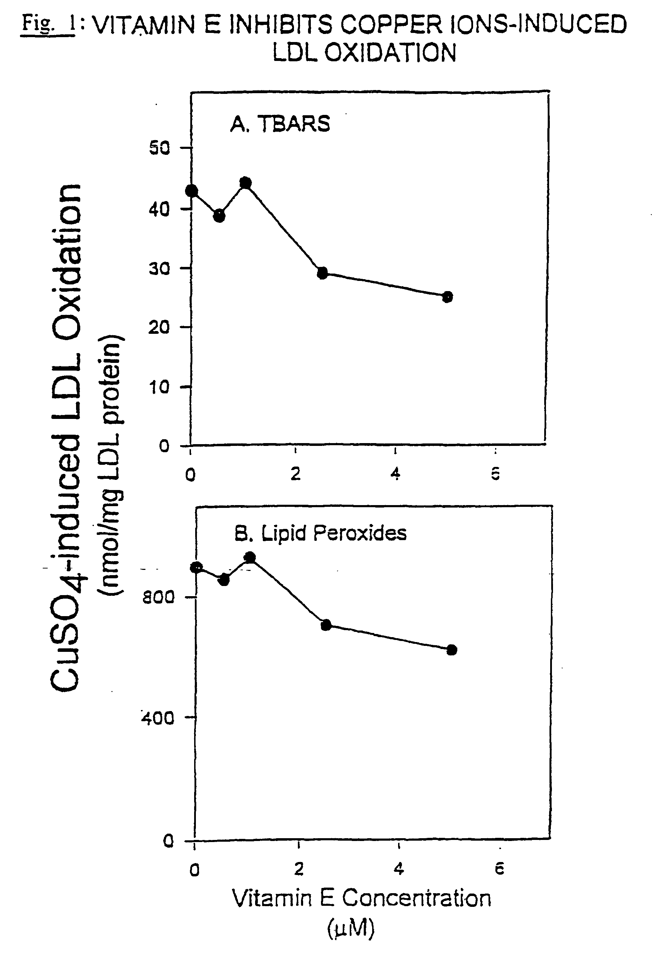

Figure 1 : Vitamin E dose dependent inhibition of $CuSO_4$ - induced LDL oxidation as measured by TBARS (A) or lipid peroxide (B) formation. Vitamin E is dissolved in tetrahydrofuran and the $IC_{50}$ for the inhibition of TBARS and lipid peroxides formation is 6 μM and 6.5 μM, respectively.

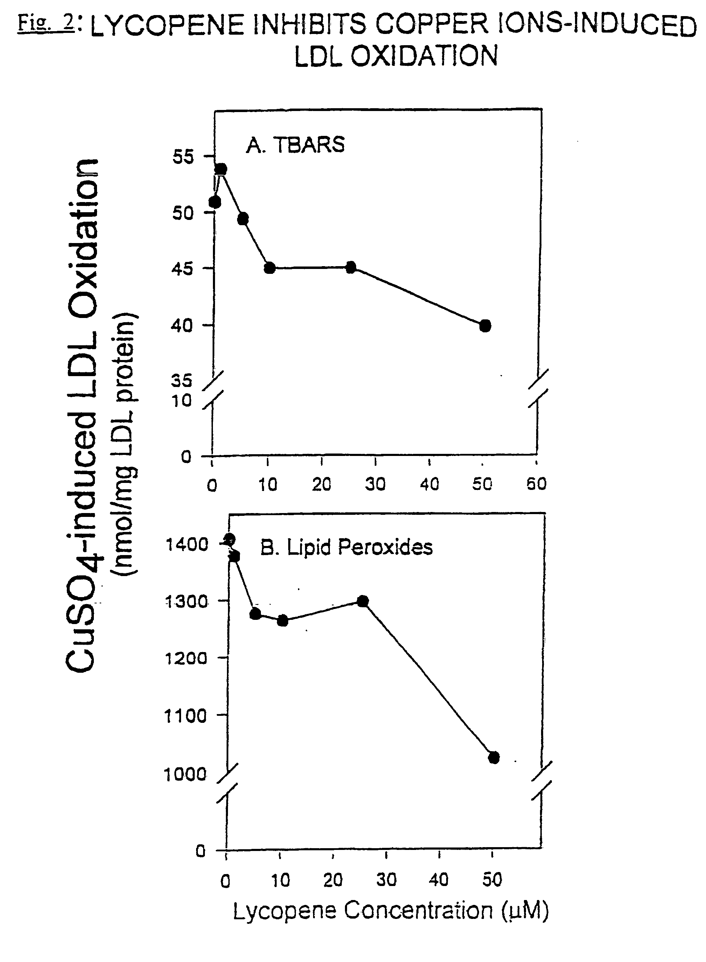

Figure 2 : Crystalline lycopene inhibition of copper-ions induced LDL oxidation. The maximal concentration used (50 μM) did not achieve 50 % inhibition, neither of TBARS nor of lipid peroxides formation.

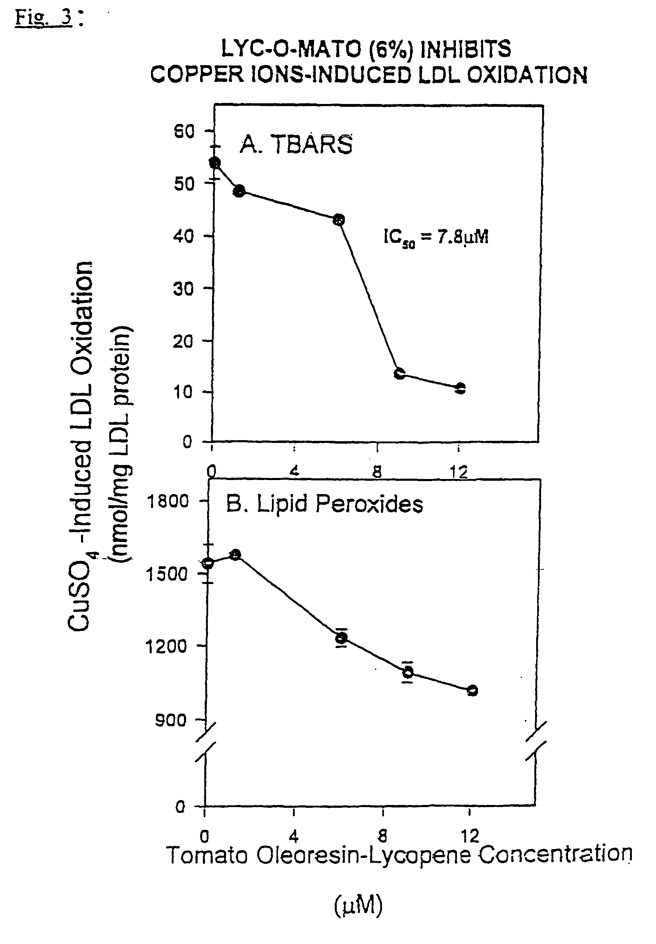

Figure 3 : LYC-O-MATO (6%) (where LYC-O-MATO is Lycored Natural Products Industries Ltd trade name for natural tomato oleoresin) inhibition of copper ions-induced LDL oxidation. The $IC_{50}$ for the inhibition of TBARS is 7.8 μM. The maximal concentration used (12 μM) did not achieve 50 % inhibition of lipid peroxides formation.

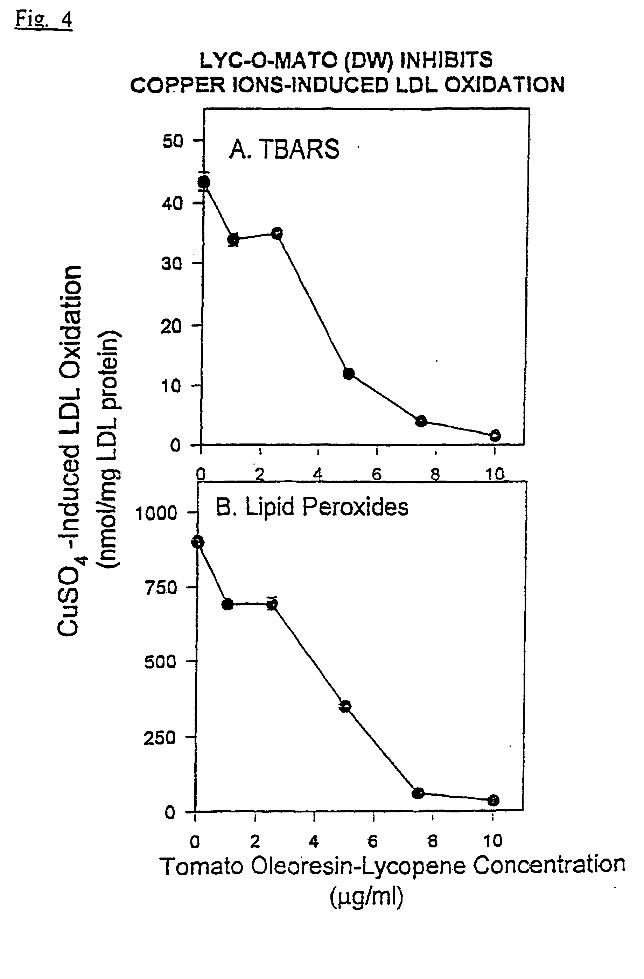

Figure 4 : Inhibition of copper ions-induced LDL oxidation of LYC-O-MATO (6%), which does not contain vitamin E {the vitamin E was extracted out and the material is designated as LYC-O-MATO (DW)}. The $IC_{50}$ for the inhibition of TBARS is 7.8 μM. The maximal concentration used (12 μM) did not achieve 50 % inhibition of lipid peroxides formation

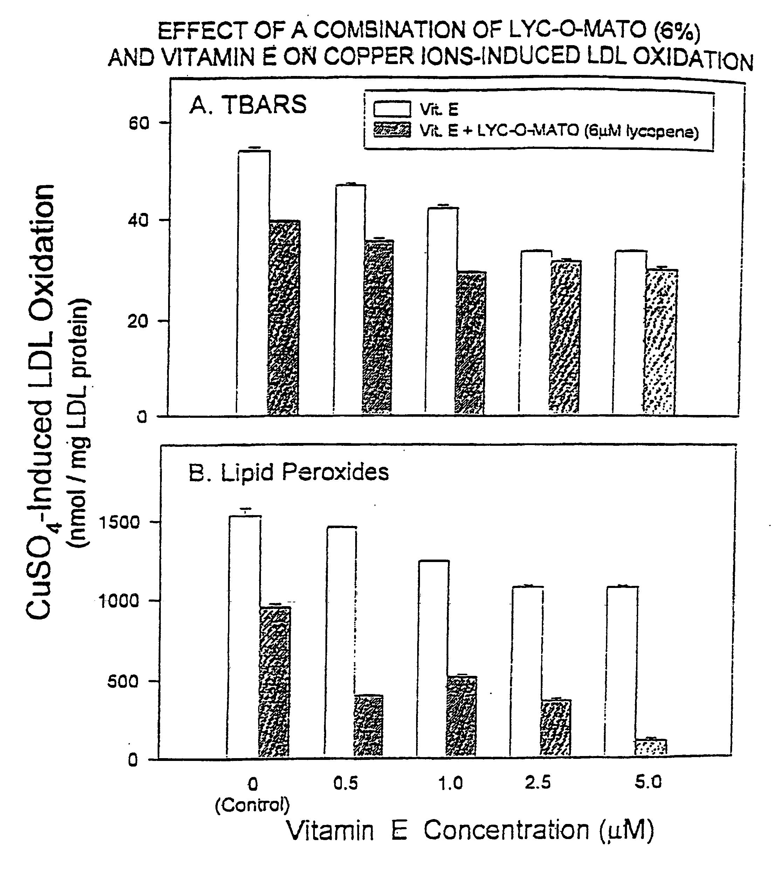

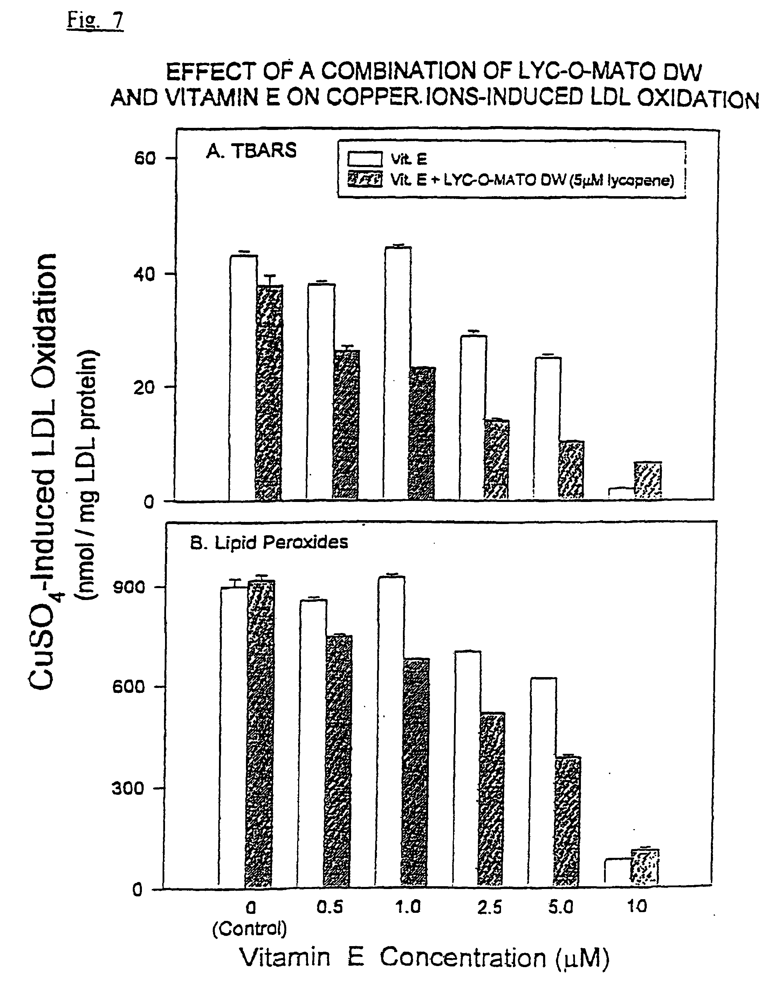

The 1 % naturally occurring vitamin E concentrates of tomato oleoresin is equal to 0.213 μM vitamin E where said oleoresin contains 6 % lycopene. Thus the true concentration of vitamin E in Figure 6 is what is listed plus 0.213 μM. Figure 5 gives data using crystalline lycopene which does not contain any vitamin E. Similarly, for Figure 7 which used tomato oleoresin where the vitamin E and other waxes were removed.

EXAMPLE 1

The Synergistic effects of a combination of vitamin E and crystalline lycopene on copper ions-induced LDL oxidation was studied. Details are shown in Table 1, affording the individual components of the Colby Equation used to calculate synergism as follows :

$$A + B - (AB/100) = \text{Expected Control}$$

where

A = Percent control of vitamin E alone
B = Percent control of lycopene alone

and synergism = $I_F/I_E$
where

$I_F$ = Actual Control in Percent
$I_E$ = Expected Control in Percent

and synergism is proven if the ratio of $I_F/I_E$ is significantly greater than 1.
The results are shown in Table 1 and Figure 5.

EXAMPLE 2

Following the method of Example 1, the synergistic effect of vitamin E and LYC-O-MATO (lycopene 6%) were determined. Results are shown in Table 1 and Figure 6.

EXAMPLE 3

Following the method of Example 1, the synergistic effect of vitamin E and vitamin E extracted LYC-O-MATO (lycopene 6%) were determined. Results are shown in Table 1 and Figure 7.

TABLE 1 :

| DATA (%) FROM EXPERIMERNTS 1 -3 FITTED TO THE COLBY EQUATION. | | | | | | | |
|---|---|---|---|---|---|---|---|
| Experiment | [a]A | [a]B | $I_E$ | $I_F$ (TBARS) | $I_F$ (LIPIDS) | $I_F/I_E$ (TBARS) | $I_F/I_E$ (LIPIDS) |
| [b]1 | 0 | 0 | 0 | 36 | 18 | infinite | infinite |
| [c]2 | 0 | [d]42 [e]32 | [d]42 [e]32 | 68 | 49 | 1.62 | 1.53 |
| [f]3 | 0 | [d]12 [e]0 | [d]12 [e]0 | 45 | 25 | 3.75 | infinite |

[a]A = vitamin E ; [a]B = lycopene.
[b] 1 μM A & 1 μM B
[c] 1 μM A & 6 μM B
[d] inhibition of TBARS
[e] inhibition of lipid peroxides
[f] 1 μM A & 5 μM B

While embodiments of the invention and experimental data have been described by way of illustration, it should be understood that the invention is not limited thereto and may be carried out with many modifications, vairations and adaptations, without departing from the scope of the claims

**Claims**

1. A method for inhibiting the oxidation of LDL in serum in vitro which comprises contacting the serum with a composition comprising a synergistic mixture of lycopene and vitamin E

2. A method in accordance with Claim 1 wherein the vitamin E concentration is in the range of 0.025 μM to 50 μM, preferably 0.1 μM to 20 μM, and most preferab!y 0.5 μM to 5 μM.

3. A method in accordance with Claim 1 wherein the concentration of lycopene is in the range of 0.025 μM to 50 μM, preferably 0.1 μM to 20 μM, and most preferably 0.5 μM to 10 μM.

4. A method in accordance with any of Claims 1 to 3 wherein the molar ratio of lycopene to vitamin E is 1:400 to 10: 1, and preferably 1:80 to 5:1.

5. A method in accordance with Claim 1 wherein the source of the lycopene is selected from the group consisting of tomato, algae, fungi, genetically modified organisms, fermentation product, synthetic lycopene and mixtures thereof.

6. A method in accordance with any of Claims 1 to 5 wherein said composition contains compounds selected from the group consisting of dietary components, additives, excipients, binding agents, coatings, preservatives and mixtures thereof.

7. A method in accordance with any of Claims 1 to 5 wherein said composition is contained in dosage forms selected from the group consisting of tablets, caplets, vegecaps and hard shell gelatin capsules.

8. Use of oleoresin derived from tomatoes for the preparation of a pharmaceutical or dietary composition for inhibiting the oxidation of LDL in serum.

9. A method for inhibiting the oxidation of LDL in serum in vitro which comprises contacting the serum with a composition comprising tomato oleoresin.

10. A method in accordance with Claim 9 wherein said composition contains compounds selected from the group consisting of dietary components, additives, excipients, binding agents, coatings, preservatives and mixtures thereof.

11. A method in accordance with any of Claims 9 to 10 wherein said composition is contained in dosage forms selected from the group consisting of tablets, caplets, vegecaps and hard shell gelatin capsules.

12. Use of a synergistic composition comprising tomato oleoresin and vitamin E for. the preparation of pharmaceutical or dietary compositions for inhibiting the oxidation of LDL in serum

13. Use in accordance with Claim 12 wherein the tomato oleoresin is selected from the group consisting of tomato oleoresin containing tocopherols (vitamin E) and tomato oleoresin wherein the tocopherols have been removed.

14. Use in accordance with Claim 12 wherein said dietary or pharmaceutical compositions contain compounds selected from the group consisting of dietary components, additives, excipieints, binding agents, coatings, preservatives and mixtures thereof.

15. Use in accordance with any of Claims 12 to 14 wherein said dietary or pharmaceutical compositions are contained in dosage forms selected from the group consisting of tablets, caplets, vegecaps, and hard shell gelatin capsules.

16. A method for inhibiting the oxidation of LDL in serum in vitro which comprises contacting the serum with a composition comprising a synergistic mixture of tomato oleoresin and vitamin E.

17. A method in accordance with Claim 1 6 wherein the tomato oleoresin is selected from the group consisting of tomato oleoresin containing tocopherols (vitamin E) and tomato oleoresin wherein the tocopherols have been removed.

18. A method in accordance with any of Claims 16 to 17 wherein said composition contains compounds selected from the group consisting of dietary components additives, excipients, binding agents, coatings, preservatives, and mixtures thereof.

19. A method in accordance with any of Claims 16 to 18 wherein said composition is contained in dosage forms selected from the group consisting of tablets, caplets, vegecaps, and hard shell gelatin capsules.

**Patentansprüche**

1. Ein Verfahren zur Inhibition der Oxidation von LDL in Serum in vitro, welches das In-Kontakt-Bringen des Serums mit einer Zusammensetzung umfasst, die eine synergistische Mischung aus Lycopin und Vitamin E umfasst.

2. Ein Verfahren in Übereinstimmung mit Anspruch 1, worin die Vitamin E-Konzentration im Bereich von 0,025 $\mu$M bis 50 $\mu$M, bevorzugt 0,1 $\mu$M bis 20 $\mu$M, und am meisten bevorzugt 0,5 $\mu$M bis 5 $\mu$M, liegt.

3. Ein Verfahren in Übereinstimmung mit Anspruch 1, worin die Konzentration von Lycopin im Bereich von 0,025 $\mu$M bis 50 $\mu$M, bevorzugt 0,1 $\mu$M bis 20 $\mu$M und am meisten bevorzugt 0,5 $\mu$M bis 10 $\mu$M, liegt.

4. Ein Verfahren in Übereinstimmung mit einem der Ansprüche 1 bis 3, worin das Molverhältnis von Lycopin zu Vitamin E 1:400 bis 10:1 und bevorzugt 1:80 bis 5:1 ist.

5. Ein Verfahren in Übereinstimmung mit Anspruch 1, worin die Quelle für Lycopin aus der Gruppe ausgewählt wird, die aus Tomaten, Algen, Pilzen, genetisch veränderten Organismen, Fermentationsprodukt, synthetischem Lycopin und Mischungen aus diesen besteht.

**6.** Ein Verfahren in Übereinstimmung mit einem der Ansprüche 1 bis 5, worin die Zusammensetzung Verbindungen enthält, die aus der Gruppe ausgewählt sind, die aus Nahrungsmittelbestandteilen, Zusatzstoffen, Bindemitteln, Bindern, Beschichtungsmaterialien, Konservierungsmitteln und Mischungen davon besteht.

**7.** Ein Verfahren in Übereinstimmung mit irgendeinem der Ansprüche 1 bis 5, worin die Zusammensetzung in Dosierformen enthalten ist, die aus der Gruppe ausgewählt werden, die aus Tabletten, Kapletten, Vegecaps und Gelatinekapseln mit harter Schale besteht.

**8.** Verwendung von aus Tomaten gewonnenem Oleoresin zur Herstellung einer pharmazeutischen oder diätetischen Zusammensetzung zur Inhibition der Oxidation von LDL in Serum.

**9.** Ein Verfahren zur Inhibition der Oxidation von LDL in Serum in vitro, welches das In-Kontakt-Bringen des Serums mit einer Zusammensetzung umfasst, die Tomatenoleoresin umfasst.

**10.** Ein Verfahren in Übereinstimmung mit Anspruch 9, worin die Zusammensetzung Verbindungen enthält, die aus der Gruppe ausgewählt sind, welche aus Nahrungsmittelbestandteilen, Zusatzstoffen, Bindemitteln, Bindern, Beschichtungsmaterialien, Konservierungsmitteln und Mischungen davon besteht.

**11.** Ein Verfahren in Übereinstimmung mit irgendeinem der Ansprüche 9 bis 10, worin die Zusammensetzung in Dosierformen enthalten ist, die aus der Gruppe ausgewählt werden, die aus Tabletten, Kapletten, Vegecaps und Gelatinekapseln mit harter Schale besteht.

**12.** Verwendung einer synergistischen Zusammensetzung, die Tomatenoleoresin und Vitamin E umfasst, für die Herstellung von pharmazeutischen oder diätetischen Zusammensetzungen zur Inhibition der Oxidation von LDL in Serum.

**13.** Verwendung in Übereinstimmung mit Anspruch 12, worin das Tomatenoleoresin gewählt ist aus der Gruppe bestehend aus Tomatenoleoresin, das Tocopherole (Vitamin E) enthält, und aus Tomatenoleoresin, aus dem die Tocopherole entfernt wurden.

**14.** Verwendung in Übereinstimmung mit Anspruch 12, worin die diätetischen oder pharmazeutischen Zusammensetzungen Verbindungen enthalten, die aus der Gruppe ausgewählt sind, welche aus Nahrungsmittelbestandteilen, Zusatzstoffen, Bindemitteln, Bindern, Beschichtungsmaterialien, Konservierungsmitteln und Mischungen davon besteht.

**15.** Verwendung in Übereinstimmung mit einem der Ansprüche 12 bis 14, worin die diätetischen oder pharmazeutischen Zusammensetzungen in Dosierformen enthalten sind, die aus der Gruppe ausgewählt werden, die aus Tabletten, Kapletten, Vegecaps und Gelatinekapseln mit harter Schale besteht.

**16.** Ein Verfahren zur Inhibition der Oxidation von LDL in Serum in vitro, welches das In-Kontakt-Bringen des Serums mit einer Zusammensetzung umfasst, die eine synergistische Mischung aus Tomatenoleoresin und Vitamin E umfasst.

**17.** Ein Verfahren in Übereinstimmung mit Anspruch 16, worin das Tomatenoleoresin aus der Gruppe ausgewählt wird, die Tomatenoleoresin einschließt, das. Tocopherole (Vitamin E) enthält, und Tomatenoleoresin, aus dem die Tocopherole entfernt wurden.

**18.** Ein Verfahren in Übereinstimmung mit einem der Ansprüche 16 bis 17, worin die Zusammensetzung Verbindungen enthält, die aus der Gruppe ausgewählt werden, welche aus Nahrungsmittelbestandteilen, Zusatzstoffen, Bindemitteln, Bindern, Beschichtungsmaterialien, Konservierungsmitteln und Mischungen davon besteht.

**19.** Ein Verfahren in Übereinstimmung mit einem der Ansprüche 16 bis 18, worin die Zusammensetzung in Dosierformen enthalten ist, die aus der Gruppe ausgewählt werden, die aus Tabletten, Kapletten, Vegecaps und Gelatinkapseln mit harter Schale besteht.

**Revendications**

1. Méthode pour inhiber l'oxydation des lipoprotéines de basse densité (LDL) dans le sérum in vitro, qui comprend la mise en contact du sérum avec une composition comprenant un mélange synergique de lycopène et de vitamine E.

2. Méthode selon la revendication 1, dans laquelle la concentration de vitamine E est dans la plage de 0,025 µM à 50 µM, de préférence de 0,1 µM à 20 µM et, de manière préférée entre toutes, de 0,5 µM à 5 µM.

3. Méthode selon la revendication 1, dans laquelle la concentration de lycopène est dans la plage de 0,025 µM à 50 µM, de préférence de 0,1 µM à 20 µM et, de manière préférée entre toutes, de 0,5 µM à 10 µM.

4. Méthode selon l'une quelconque des revendications 1 à 3, dans laquelle le rapport molaire du lycopène à la vitamine E est de 1/400 à 10/l, et de préférence de 1/80 à 5/l.

5. Méthode selon la revendication 1, dans laquelle la source du lycopène est choisie dans le groupe comprenant tomate, algues, champignons, organismes génétiquement modifiés, produit de fermentation, lycopène de synthèse et les mélanges de ceux-ci.

6. Méthode selon l'une quelconque des revendications 1 à 5, dans laquelle ladite composition contient des composés choisis dans le groupe comprenant composants diététiques, additifs, excipients, liants, enrobages, conservateurs et les mélanges de ceux-ci.

7. Méthode selon l'une quelconque des revendications 1 à 5, dans laquelle ladite composition est contenue dans des formes galéniques choisies dans le groupe constitué par les comprimés, les caplets, les capsules végétales et les capsules à enveloppe dure de gélatine.

8. Utilisation d'oléorésine dérivée de tomates pour la préparation d'une composition pharmaceutique ou diététique pour inhiber l'oxydation des LDL dans le sérum.

9. Méthode pour inhiber l'oxydation des LDL dans le sérum in vitro, qui comprend la mise en contact du sérum avec une composition comprenant de l'oléorésine de tomate.

10. Méthode selon la revendication 9, dans laquelle ladite composition contient des composés choisis dans le groupe comprenant composants diététiques, additifs, excipients, liants, enrobages, conservateurs et les mélanges de ceux-ci.

11. Méthode selon l'une quelconque des revendications 9 à 10, dans laquelle ladite composition est contenue dans des formes galéniques choisies dans le groupe constitué par les comprimés, des caplets, les capsules végétales et les capsules à enveloppe dure de gélatine.

12. Utilisation d'une composition synergique comprenant de l'oléorésine de tomate et de la vitamine E pour la préparation de compositions pharmaceutiques ou diététiques pour inhiber l'oxydation des LDL dans le sérum.

13. Utilisation selon la revendication 12, dans laquelle l'oléorésine de tomate est choisie dans le groupe constitué par l'oléorésine de tomate contenant des tocophérols (vitamine E) et l'oléorésine de tomate de laquelle les tocophérols ont été enlevés.

14. Utilisation selon la revendication 12, dans laquelle lesdites compositions diététiques ou pharmaceutiques contiennent des composés choisis dans le groupe comprenant composants diététiques, additifs, excipients, liants, enrobages, conservateurs et les mélanges de ceux-ci.

15. Utilisation selon l'une quelconque des revendications 12 à 14, dans laquelle lesdites compositions diététiques ou pharmaceutiques sont contenues dans des formes galéniques choisies dans le groupe constitué par les comprimés, les caplets, les capsules végétales et les capsules à enveloppe dure de gélatine.

16. Méthode pour inhiber l'oxydation des LDL dans le sérum in vitro, qui comprend la mise en contact du sérum avec une composition comprenant un mélange synergique d'oléorésine de tomate et de vitamine E.

**17.** Méthode selon la revendication 16, dans laquelle l'oléorésine de tomate est choisie dans le groupe constitué par l'oléorésine de tomate contenant des tocophérols (vitamine E) et l'oléorésine de tomate de laquelle les tocophérols ont été enlevés.

**18.** Méthode selon l'une quelconque des revendications 16 à 17, dans laquelle ladite composition contient des composés choisis dans le groupe comprenant composants diététiques, additifs, excipients, liants, enrobages, conservateurs et les mélanges de ceux-ci.

**19.** Méthode selon l'une quelconque des revendications 16 à 18, dans laquelle ladite composition est contenue dans des formes galéniques choisies dans le groupe'constitué par les comprimés, les caplets, les capsules végétales et les capsules à enveloppe dure de gélatine.

Fig. 1: VITAMIN E INHIBITS COPPER IONS-INDUCED LDL OXIDATION

Fig. 2: LYCOPENE INHIBITS COPPER IONS-INDUCED LDL OXIDATION

Fig. 3 :

LYC-O-MATO (6%) INHIBITS
COPPER IONS-INDUCED LDL OXIDATION

14

Fig. 4

LYC-O-MATO (DW) INHIBITS
COPPER IONS-INDUCED LDL OXIDATION

EFFECT OF A COMBINATION OF LYCOPENE
AND VITAMIN E ON COPPER IONS-INDUCED LDL OXIDATION

EFFECT OF A COMBINATION OF LYC-O-MATO (6%)
AND VITAMIN E ON COPPER IONS-INDUCED LDL OXIDATION

A. TBARS

B. Lipid Peroxides

Fig. 7

EFFECT OF A COMBINATION OF LYC-O-MATO DW
AND VITAMIN E ON COPPER IONS-INDUCED LDL OXIDATION